# EUROPEAN PATENT SPECIFICATION

(11) **EP 4 197 428 B1**
(45) Date of publication and mention of the grant of the patent: **20.05.2026**
(21) Application number: 22212313.5
(22) Date of filing: 08.12.2022
(51) Int. Cl.: A61B 3/113, A61F 9/008

(54) **OPTHALMOLOGICAL TREATMENT DEVICE FOR DETERMINING A ROTATION ANGLE OF AN EYE**
OPTHALMOLOGISCHE BEHANDLUNGSVORRICHTUNG ZUR BESTIMMUNG EINES DREHWINKELS EINES AUGES
DISPOSITIF DE TRAITEMENT OPHTALMOLOGIQUE POUR DÉTERMINER UN ANGLE DE ROTATION D'UN OEIL

(30) Priority: 20.12.2021 CH 0707462021
(43) Date of publication of application: 21.06.2023
(73) Proprietor: Ziemer Ophthalmic Systems AG, 2562 Port (CH)
(72) Inventor: MÜLLER, Fabian, 2502 Biel (CH); RATHJEN, Christian, 28197 Bremen (DE); STEINLECHNER, Michael, 8006 Zürich (CH)
(74) Representative: Rentsch Partner AG

(56) References cited:
- WO-A1-2006/060323
- WO-A2-01/89373
- US-A1- 2003 223 037
- US-A1- 2004 143 244
- US-A1- 2014 049 750
- US-A1- 2015 105 759
- US-A1- 2017 007 446
- MORLEY DUSTIN ET AL: "Computing Cyclotorsion in Refractive Cataract Surgery", IEEE TRANSACTIONS ON BIOMEDICAL ENGINEERING, IEEE, USA, vol. 63, no. 10, 1 October 2016 (2016-10-01), pages 2155 - 2168, XP011623527, ISSN: 0018-9294, [retrieved on 20160921], DOI: 10.1109/TBME.2015.2511759

## Description

### Field of the Invention

The present disclosure relates to an ophthalmological treatment device for determining a rotation angle of an eye of a person.

### Background of the Invention

Treatment of a human eye, for example using laser ablation, depends critically on a correct alignment between the eye and the laser such that the correct areas of the eye are treated. The basis of the treatment is defined by a treatment model, which is used to control the laser during treatment. The treatment model is established based on measurements of the eye taken using a diagnostic device.

While setting up the laser for treatment, and also during treatment, it is important to detect an actual position and an actual direction of gaze of the eye such that the laser performs ablation according to the treatment model. In addition, a rotation of the eye, including, for example, a torsion of the eye about an axis (known as cyclotorsion) is important to account for, in particular for astigmatic eyes.

Some eyes experience varying degrees of cyclotorsion when the axis moves from a horizontal orientation, as is typical when the person is upright, to an inclined position, for example when the person is lying down.

Further, a rotation of the head can also lead to a rotation of the eye.

This presents a challenge during eye treatment, as treatment of the eye, for example using laser ablation, must account for the cyclotorsion for best results, in particular for eyes with astigmatism. This is because the treatment model is established based on measurements of the eye taken using a diagnostic device with the person in an upright position, whereas the treatment is usually performed with the person in a supine position

Known methods for measuring and accounting for a rotation of the eye, in particular a cyclotorsion, include manually marking the eyeball of the person when the person is in the upright position and realigning the treatment model according to the mark.

US8708488B2 describes a method comprising comparing images recorded before the surgery with images recorded during surgery in order to generate a marker which represents a target orientation. An orientation is determined such as to compensate for a rotation of the eye, using image comparison, which is directed to structures of the sclera of the eye, in particular structures of blood vessels.

US7331767B2 describes a method for aligning diagnostic and therapeutic iris images, via iris pattern recognition, for effecting more accurate laser treatment of the eye. In an example, an iris landmark is identified which is tracked by having a sequential plurality of diagnostic iris images of varying pupil size such that the iris landmark can be tracked between the two images. The aligned, constricted pupil diagnostic image can then be aligned with a constricted pupil treatment image and the ablation pattern rotated accordingly. Limbal edge detection is used in the diagnostic images to provide pupil center translation information for translational alignment of the laser treatment. Iris pattern recognition is used by identifying iris patterns using markers (artificial) or landmarks (natural).

US9936866B2 describes a method of receiving a first optical data set of an eye with a pupil having a first pupil size; and receiving a second optical data set of the eye with the pupil having a second pupil size. A pseudo-rotation related to a pupil size change is determined, a measured cyclotorsion is received, an actual cyclotorsion is calculated from the measured cyclotorsion and the pseudo-rotation, and a laser treatment is adjusted according to the actual cyclotorsion.

US7044602B2 describes methods and systems for tracking a position and torsional orientation of a person's eye, comprising selecting at least one marker on the iris of the eye in the first image. A corresponding marker is located on the iris in the second image. The first image of the eye and the second image of the eye are registered by substantially matching a common reference point in the first and second images and matching the marker on the iris of the image of the first eye and the marker on the iris of the image of the second eye.

US2014049750A1 discloses a system for registering and tracking the position of a person's eye, in particular for refractive ophthalmic surgery. According to embodiments, the system is structured such that eye images containing at least the iris and the pupil of the eye are made at a first wavelength of light and that eye images containing scleral blood vessels are made at a different second wavelength of light.

US2015105759A1 discloses methods and systems for determining a plurality of correlation measures and taking the cyclotorsion angle that gives the strongest correlation.

These and other methods have the drawbacks such as requiring both the diagnostic image(s) and the images recorded during treatment to be taken under known conditions (e.g. lighting conditions), using known equipment, and/or that they require manual intervention such as manually marking the sclera of the eye. Other drawbacks include relying on particular features of the eye to be detectable, for example the blood-vessels in the sclera, requiring multiple diagnostic images and/or images taken directly before treatment, or requiring a computation involving an iterative optimization to find the cyclotorsion angle.

### Summary of the Invention

It is an object of embodiments disclosed herein to provide an ophthalmological treatment device for determining a rotation angle of an eye of a person which overcomes one or more of the disadvantages of the prior art.

In particular, it is an object of embodiments disclosed herein to provide an ophthalmological treatment device and method for determining a rotation angle of an eye of a person.

According to embodiments of the present disclosure, the above-mentioned objects are achieved by an ophthalmological treatment device comprising a processor and a camera for determining a rotation of an eye of a person. The processor is configured to receive a reference image of the eye of the person. The reference image is an image of the eye of the person, recorded at a prior time by a separate diagnostic device. The reference image having been taken while the person was in an upright position, e.g. sitting on a chair. The processor is configured to record, using the camera, a current image of the eye of the person in a reclined position, e.g. sitting back or lying horizontally. The processor is configured to determine a rotation angle of the eye, using a direct solver, by comparing the reference image to the current image.

In an embodiment, the direct solver includes a software application, an algorithm, and/or a function. The direct solver includes, depending on the implementation, one or more software libraries and/or data-sets. Depending on the embodiment, the direct solver is implemented on the processor of the ophthalmological treatment device and/or on a computer connected to the ophthalmological treatment device, in particular connected via one or more communication networks.

In an embodiment, the processor is further configured to control the ophthalmological treatment device using the rotation angle.

In an embodiment, the processor is further configured to rotate a treatment pattern of a laser by the rotation angle, which treatment pattern is configured for the eye of the person. In a preferred embodiment, the rotation angle includes a cyclotorsion angle.

According to the invention, the direct solver is configured to determine the rotation angle using a pre-defined number of computational operations, preferably within a pre-defined number of milliseconds.

In an embodiment, the processor, in particular the direct solver, is configured to determine the rotation angles of the eye by: identifying one or more non-local features of the reference image and non-local features of the current image, and matching the one or more identified non-local features of the reference image to the one or more identified non-local features of the current image, respectively. Non-local features refer to features (e.g. patterns or structures) in an image which exist at scales larger than local features. Local features, by way of contrast to non-local features, include points or edges, pixels (or small groups thereof) of a particular colour, having a particular contrast gradient. Local features may also refer to a sub-sample of the image having a particular texture, colour, or intensity. Local features, as opposed to non-local features, are distinctive, in that they are either present in a distinctive sub-sample of an image, or not present. Further, local features, as opposed to non-local features, are localizable in that they have a unique location in the image associated with the feature. Non-local features, however, refer to features which do not have a defined location. These non-local features may, however, have properties such as an orientation and/or a scale. Examples of non-local features include, for example, an iris colour, radial stripes in an iris, and a texture of an eye.

In an embodiment, the processor is configured to determine the rotation angle of the eye by applying a pre-determined sequence of signal processing filters to both the entire reference image and to the entire current image.

In an embodiment, the pre-determined sequence of signal processing filters comprises one or more of: a convolutional operator, an activation function, or a pooling function.

In an embodiment, the pre-determined sequence of signal processing filters is part of a neural network.

In an embodiment, the neural network is trained to determine the rotation angle using supervised learning. The neural network is trained using a training dataset. The training dataset comprises a plurality of training reference images, a plurality of corresponding training current images, and a plurality of corresponding pre-defined rotation angles, respectively. Each training reference image has an associated training current image and a pre-defined rotation angle. Thereby, the neural network is trained to generate a rotation angle when given a training reference image and a training current image as an input, the rotation angle generated being within a small error margin of the actual pre-defined rotation angle.

In an embodiment, the ophthalmological treatment device comprises a first neural network and a second neural network both having identical architecture and parameters. The first neural network is configured to receive the reference image as an input and to generate a reference image output vector. The second neural network is configured to receive the current image as an input and to generate a current image output vector. The processor is configured to determine the rotation angle using the reference image output vector, the current image output vector, and a distance metric.

According to the invention, the processor, in particular the direct solver, is configured to determine the rotation angle by generating a reference image output vector using the reference image and the pre-determined sequence of signal processing filters. The processor is configured to determine the rotation angle by generating a current image output vector using the current image and the pre-determined sequence of signal processing filters. The processor is configured to determine the rotation angle by determining a distance between the reference image output vector and the current image output vector using a distance metric.

In an embodiment, the processor is further configured to pre-process the images. Pre-processing comprises: detecting the iris of the eye in the reference image and/or the current image, in particular detecting an edge between the iris and the pupil of the eye; detecting scleral blood vessels of the eye in the reference image and/or the current image; detecting the retina of the eye in the reference image and/or the current image; identifying a covered zone in the reference image, which covered zone is a part of the eye covered by the eyelid; unrolling the reference image and/or the current image using a polar transformation; rescaling the reference image and/or the current image according to a detected pupil dilation in the reference image and/or the current image; image correcting the reference image and/or the current image, in particular by matching an exposure, a contrast, or a color; and/or resizing the reference image and/or the current image such that the reference image and the current image have a matching size.

In an embodiment, the processor is configured to receive a color reference image and/or an infrared reference image. The processor is configured to record, using the camera, a color current image and/or an infrared current image. The processor is, in an embodiment, configured to determine the rotation angle using the color reference image and/or an infrared reference image and the current image and/or an infrared current image, respectively, using the direct solver.

In an embodiment, the processor is further configured to transmit the current image to a further ophthalmological treatment device 1B.

In an embodiment, the processor is configured to determine whether the reference image and the current image are both images of the same eye, in particular of the same eye of the person.

In addition to an ophthalmological treatment device, the present disclosure also relates to a method for determining a rotation of an eye of a person comprising a processor of an ophthalmological treatment device performing the step of receiving a reference image of the eye of the person, the reference image having been recorded of the eye of the person in an upright position by a separate diagnostic device. The method comprises recording, using the camera, a current image of the eye of the person in a reclined position. The method comprises determining a rotation angle of the eye by comparing the reference image to the current image, using a direct solver.

The method comprises further steps as set forth in claim 9.

In an embodiment, the method further comprises rotating a treatment pattern of a laser about the rotation angle, which treatment pattern is configured for the eye of the person. In an embodiment, determining the rotation angle of the eye comprises applying a pre-determined sequence of signal processing filters to both the entire reference image and the entire current image.

In an embodiment, the pre-determined sequence of signal processing filters comprises one or more of: a convolutional operator, an activation function, or a pooling function.

In an embodiment, the pre-determined sequence of signal processing filters is part of a neural network.

In an embodiment, the neural network is trained to determine the rotation angle using supervised learning and a training dataset, wherein the training dataset comprises a plurality of training reference images, a plurality of corresponding training current images, and a plurality of corresponding pre-defined rotation angles, respectively.

In an embodiment, the method comprises using a first neural network and a second neural network both having an identical architecture and identical parameters. The first neural network is configured to receive the reference image as an input and generate a reference image output vector. The second neural network is configured to receive the current image as an input and to generate a current image output vector. Determining the rotation angle comprises using the reference image output vector, the current image output vector, and a distance metric.

In addition to an ophthalmological device and a method, the present disclosure also relates to a computer program product comprising a non-transitory computer-readable medium having stored thereon computer program code for controlling a processor of an ophthalmological device to receive a reference image of an eye of a person, the reference image having been recorded of the eye of the person in an upright position by a separate diagnostic device. The program code controls the processor to record, using a camera of the ophthalmological device, a current image of the eye of the person in a reclined position. The program code controls the processor to determine a rotation angle of the eye by comparing the reference image to the current image using a direct solver.

The program code further controls the processor as set forth in claim 13.

### Brief Description of the drawings

The herein described disclosure will be more fully understood from the detailed description given herein below and the accompanying drawings which should not be considered limiting to the disclosure described in the appended claims. The drawings in which:
- Figure 1:: shows a block diagram illustrating schematically an ophthalmological treatment device for treating eye tissue with a pulsed laser beam, comprising a processor and a camera for determining a rotational angle of an eye of a person;
- Figure 2:: shows a schematic cross-sectional view of a patient interface of the ophthalmological treatment device with a curved internal space for receiving a corneal region of the eye and suction elements for fixing the patient interface on the eye;
- Figure 3a:: shows a diagnostic device configured to record a reference image of an eye of a person in an upright position;
- Figure 3b:: shows an ophthalmological treatment device configured to record a current image of an eye of a person in a supine position;
- Figure 4:: shows the cornea of an eye of the person;
- Figure 5:: shows a schematic view of an incision surface in an eye and a treatment pattern.
- Figure 6a:: shows a schematic view of an incision surface in an eye tissue region which is unrotated with respect to the central axis of the patient interface.
- Figure 6b:: shows a schematic view of an incision surface in an eye tissue region which is rotated with respect to the central axis of the patient interface.
- Figure 7:: show a flow diagram illustrating a sequence of steps for determining a rotation angle of an eye;
- Figure 8:: shows a flow diagram illustrating a sequence of steps for determining a rotation angle of an eye using a direct solver;
- Figure 9:: shows a block diagram illustrating a direct solver configured to determine a rotation angle of an eye;
- Figure 10:: shows a block diagram illustrating a direct solver comprising a neural network;
- Figure 11:: shows a block diagram illustrating a direct solver comprising two neural networks; and
- Figure 12:: shows a block diagram illustrating how the neural network is trained.

### Detailed Description of embodiments

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

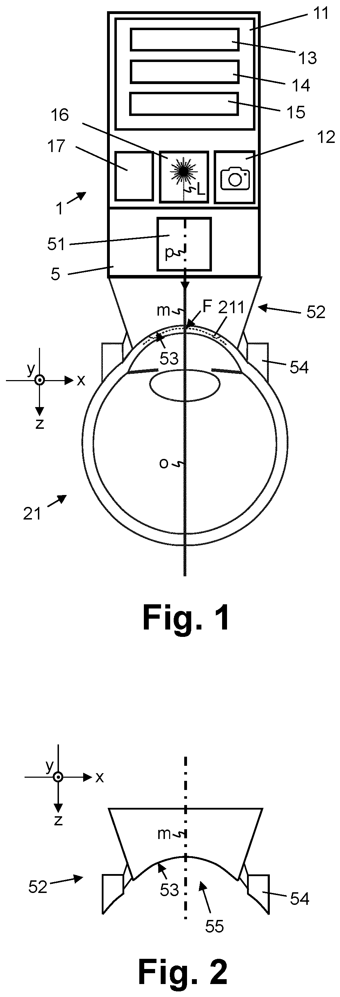

**Figure** 1 shows an ophthalmological treatment device 1 for treating eye tissue by means of laser pulses, for example the cornea 211 or another tissue of an eye 21 of a person 2.

As is schematically represented in **Figure** 1, the ophthalmological treatment device 1 comprises a control module 11, a measuring device 12, a laser source 16, a scanner system 17, and an application head 5 having focusing optics 51 and a patient interface 52. The control module has one or more processors 13, a memory 14, and a communication interface 15.

The laser source 16 is configured to generate a pulsed laser beam L. The laser source 16 comprises in particular a femtosecond laser for generating femtosecond laser pulses, which have pulse widths of typically from 10 fs to 1000 fs (1 fs = 10⁻¹⁶ s). The laser source 3 is arranged in a separate housing or a common housing with the focussing optics 51.

The scanner system 17 is configured to steer the pulsed laser beam L delivered by the laser source 16 by means of the focussing optics 51 in the eye tissue 211 onto treatment points F on a treatment pattern t (comprising a laser trajectory). In an embodiment, the scanner system 17 comprises a divergence modulator for modulating the focal depth, or the treatment height, in the projection direction along the projection axis p. The scanner system 17 comprises, for example, a galvanoscanner or a piezo-driven scanner. Depending on the embodiment, the scanner 17 additionally comprises one or more deflecting mirrors, one or more resonant mirrors, or one or more oscillating mirrors, which are for example piezo-driven, or MEM (Micro-Electromechanical), or the scanner system 17 comprises an AOM (Acousto-Optical Modulator) scanner or an EOM (Electro-Optical Modulator) scanner.

As is schematically represented in **Figure** 1, the application head 5 comprises focussing optics 51 and a patient interface 52. The application head 5 can be placed onto the eye 21 and fixed on the eye 21 by means of the patient interface 52. For the sake of simplicity, only the application head 5 applied and fixed on the eye 21 is respectively represented in **Figure** 2, without reproducing the further modules of the ophthalmological device 1 which are represented in Figure 1.

The focussing optics 51 are configured to focus the pulsed laser beam L, or its laser pulses, onto the treatment points F inside the eye tissue 211 for the pointwise tissue disruption. The focussing optics 51 comprise a lens system having one or more optical lenses.

Depending on the embodiment, the focussing optics 51 comprise one or more movable lenses and/or a drive for moving the entire focussing optics 51 in order to set and adjust the focal depth, or the treatment height, in the projection direction along the projection axis p. In a further embodiment, a divergence modulator is provided in the beam path between the laser source 16 and the scanner system 17.

For the treatment and incision of incision surfaces C, C' which have a lateral component in the x/y treatment plane normal to the projection direction which is comparatively larger than the depth component in the projection direction along the projection axis p, the scanner system 17 is configured to displace the treatment points F, onto which the laser pulses are focussed, with a higher scan speed on the treatment pattern t, t' in relation to the focus adjustment speed of the focussing optics 51.

Although reference is made to the incision according to an incision surface C, C', the treatment pattern t, t' also relates to treatment of the eye by surface ablation, depending on the embodiment.

As is schematically represented in **Figures 1** and **2****,** the patient interface 52 comprises one or more holding elements, for example suction elements 54, for fixing the patient interface 52 on the eye 21. The suction element 54 is, for example, configured as a suction ring. As is schematically represented in Figure 2, the patient interface 52 comprises a curved internal space 55 for receiving a corneal region 211 of the eye 21. The curved internal space 55 is symmetrical with respect to the central axis m of the patient interface 52. The curved internal space 55 is formed by a contact surface 53 of the patient interface 52. In the applied state, the contact surface 53 of the patient interface 52 makes contact with the outer surface of the cornea 211 of the eye 21. Depending on the embodiment, the patient interface 52 is connected securely or removably to the focussing optics 51.

In an embodiment, the patient interface 52, in particular the contact surface 53, has a flattened central section and the eye 21 is removably attached to the patient interface 52 by applanation, in which the normally curved surface of the cornea 211 is held in a flattened state against the contact surface 53 of the patient interface 52 by the suction element 54.

In an embodiment, the patient interface 52 does not have a contact surface 53 or a suction element 54 and the treatment takes place without fixing the eye 21. Specifically, the patient interface 52 and the eye 21 are separated by an air gap of several centimetres, for example.

As is schematically represented in **Figure** 1, the ophthalmological device 1 comprises a control module 11 for controlling the ophthalmological device 1. The control module 11 is configured to control the laser source 15, the optical functional module of the scanner system 16, the focussing optics 51, and the measuring device 12 described below. The control module 11 embodies a programmable control device and comprises, for example, one or more processors 13, and one or more memories 14 (comprising volatile and/or non-volatile memory, e.g., random-access memory and/or flash memory) having stored thereon program code, data, as well as programmed software modules for controlling the processors 11, and/or other programmable circuits or logic units such as ASICs (Application-Specific Integrated Circuits), for example GPUs (graphics processing units) and TPUs (tensor processing units). The control module 11 is connected to the various components of the ophthalmological device 1 using the communication interface 15. In particular, the memory 14 is configured to store control data relating to a treatment pattern t used to treat an eye 21 of a person 2.

The communication interface 15 is further configured for data communication with one or more external devices. Preferably, the communication interface 15 comprises a network communications interface, for example an Ethernet interface, a WLAN interface, and/or a wireless radio network interface for wireless and/or wired data communication using one or more networks, comprising, for example, a local network such as a LAN (local area network), and/or the Internet.

The skilled person is aware that at least some of the steps and/or functions described herein as being performed on the processor 11 of the ophthalmological device 1 may be performed on one or more auxiliary processing devices connected to the processor 11 of the ophthalmological device 1 using the communication interface 15. The auxiliary processing devices can be co-located with the ophthalmological device 1 or located remotely, for example on a remote server computer.

The skilled person is also aware that least some of the data associated with the program code (application data) or data associated with a particular patient (patient data) and described as being stored in the memory 14 of the ophthalmological device 1 may be stored on one or more auxiliary storage devices connected to the ophthalmological device 1 using the network interface 15.

The ophthalmological device 1 optionally includes a user interface comprising, for example, one or more user input devices, such as a keyboard, and one or more output devices, such as a display. The user interface is configured to receive user inputs from an eye treatment professional, in particular based on, or in response to, information displayed to the eye treatment professional using the one or more output devices.

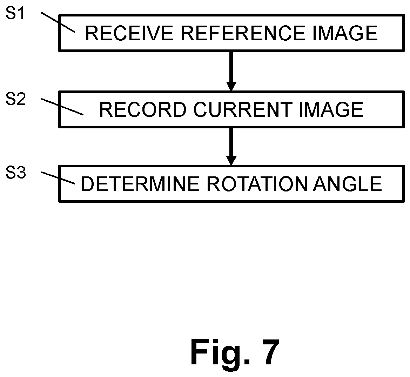

As is schematically represented in **Figure 1****,** the ophthalmological device 1 comprises a measuring device 12, for example an imaging measuring device, for example a camera 221 (e.g. comprising a CMOS or CCD chip) configured to record one or more color and/or infrared images, or an interferometric measuring device, for example an OCT (Optical Coherence Tomography) system, which is optically coupled into the beam path of the scanner system 17 towards the focussing optics 51 . The measuring device 12 is configured to record a current image 121 of the eye 2 and/or record current interferometric data of the eye 2. The measuring device 12 is connected to the control module 12. As is explained below in more detail, in particular with reference to **Figures 7** to **11****,** the control module 12 is configured to determine a rotation angle of the eye 21, on the basis of the current image 121 and/or on the basis of the current interferometric data, in addition to reference data of the eye comprising a reference image 31 and/or reference interferometric data. Throughout this disclosure, reference will be made primarily to a reference image 31 and a current image 121 for brevity, however current interferometric data and reference interferometric data is to be included unless it is explicitly excluded.

The control module 12, more particularly the processor, determines a rotation angle **θ** of the eye 21 using a direct solver S, in particular a rotation angle **θ** in relation to the central axis m of the patient interface 52. The direct solver S is stored in the memory 14.

The rotation angle **θ** is an angle of rotation of the eye 21 about an axis. The axis is, for example, parallel to a central axis m of the patient interface 52. **Figures 1** and **2** represent situations in which the eye 21 is aligned coaxially with respect to the central axis m of the patient interface 52, such that a central axis o of the eye 21 is coincident with the central axis m of the patient interface 52.

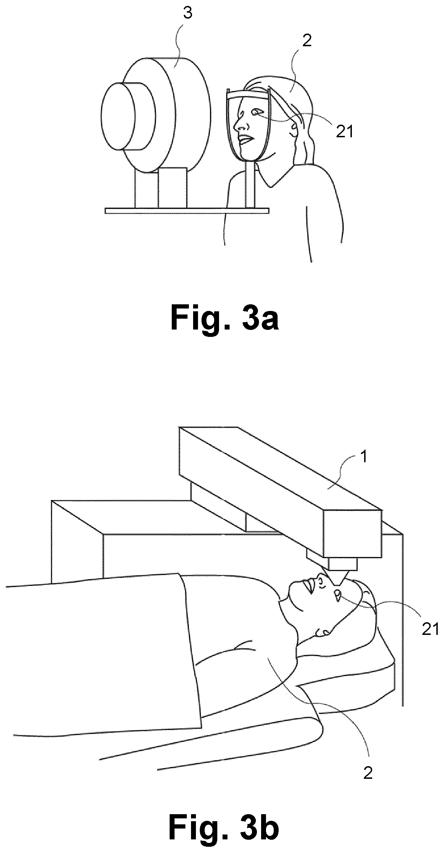

As described in more detail with reference to **Figures 3a** and **3b** below, the eye 21 of the person 2 rotates about an axis when the person 2, in particular the head of the person 2, moves from a substantially upright position to an inclined position (for example a horizontal position). This rotation is caused, for example, by cyclotorsion. The rotation can also be caused, for example, by the head of the person 2 being rotated with respect to the patient interface 52.

As described herein in more detail, the treatment pattern t is not rotationally symmetric for each eye, owing to some persons 2 having a degree of astigmatism. Therefore, it is important to account for any rotation of the eye 2, in particular by rotating the treatment pattern t according to the rotation angle **θ**.

**Figure 3a** shows an illustration of a person 2 having a reference image 31 and/or reference interferometric data being recorded by a diagnostic device 3. The person 2, in particular the head of the person 2, is upright, such that the eye 21 of the person is looking in a substantially horizontal direction. The reference image 31 and/or reference interferometric data is recorded prior to treatment. In particular, the reference image 31 and/or reference interferometric data, along with other data measured by the diagnostic device 3 is used to generate a treatment pattern t specific to the eye 21 of the person, for example to correct an imperfection in the curvature of the eye causing blurry vision. The treatment pattern t in particular corrects for astigmatism.

The diagnostic device 3 is configured to record and store the reference image 31 and/or reference interferometric data. The reference image 31 and/or reference interferometric data is then provided to the ophthalmological treatment device 1. For example, the reference image 31 and/or reference interferometric data is transmitted to the ophthalmological treatment device 1 using a data communications network, for example the Internet. Alternatively, the reference image 31 and/or reference interferometric data is stored to a portable data carrier which is then connected to the ophthalmological treatment device 1 .

**Figure 3b** shows an illustration of a person 2 lying in a reclined or a substantially horizontal position for eye treatment. The person 2, in particular the head of the person, is oriented such that the eye 21 looks upwards in a substantially vertical direction. Due to cyclotorsion or other causes, such as a rotation of the head, the eye 21 may be rotated about the rotation angle.

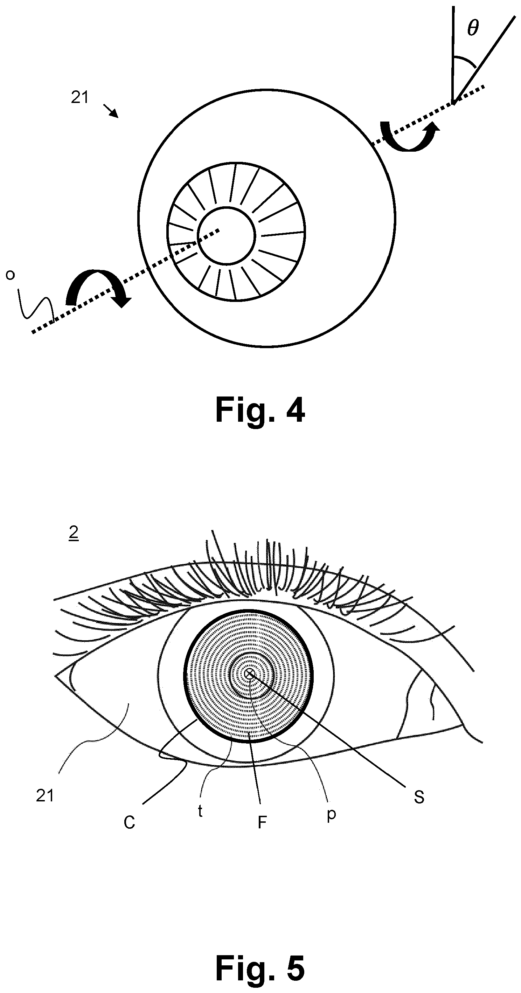

**Figure 4** shows an illustration of an eye 21 having a central axis o about which the eye 21 rotates by the rotation angle **θ**. In particular, some people experience cyclotorsion which is a rotation of the eye 21 as the head of the person tilts backwards.

**Figure 5** shows a treatment pattern t for incising the incision surface C. The treatment pattern t is curved and extends around the projection axis p of the focussing optics 51. In Figure 5, the projection axis p aligns with the central axis of the eye o (not shown), however, this is not necessarily the case. The treatment pattern t includes a helical laser trajectory which extends on the incision surface C starting from the apex S of the incision surface C and has a continual slope with a monotonic change in the treatment height in the direction of the projection axis p. In this case, the apex S of the incision surface C is the highest point of the incision surface C in respect of the direction of the projection axis p. The treatment pattern t furthermore has a distance from the projection axis p that becomes greater with a decreasing treatment height, or increasing treatment depth. The person skilled in the art will understand that the incision process by scanning the treatment pattern t may be carried out starting from its deepest point in the eye 6 towards its apex S, or vice versa. Depending on the embodiment and configuration, the treatment pattern t comprises one or more mutually separated or connected pattern sections. The pattern sections have a circular, elliptical, parabolic, hyperbolic, helical and/or spline shape. The treatment pattern t for incising an incision surface C is defined by stored control data. On the basis of the control data, the control module 11 controls the laser source 16, the scanner system 17 and/or the focussing device 51 in order to incise the incision surface C defined by the control data by scanning the treatment pattern t by means of the pulsed laser beam L. The treatment pattern t, specifically the pattern sections, comprises a plurality of treatment points F onto which the pulse laser beam L is directed.

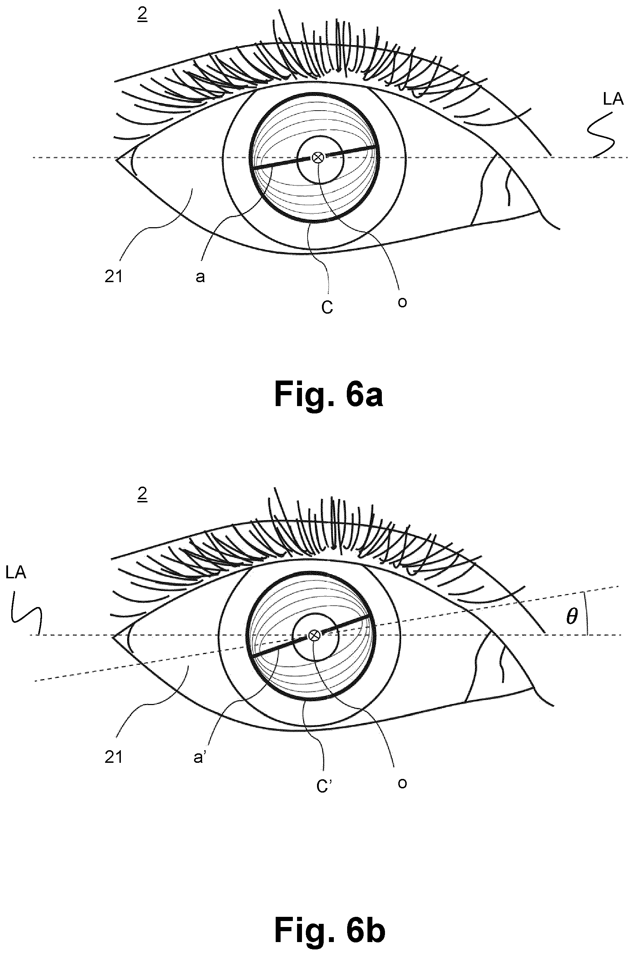

**Figure 6a** shows an illustration of an incision surface C in the eye tissue 211 of the eye 21, in particular the cornea, which is asymmetric, e.g. it has an asymmetric thickness profile, with respect to the central axis m of the patient interface 52 when the central axis m of the patient interface 52 is aligned coaxially with respect to the projection axis p of the focussing optics 51. The thickness of the incision surface C varies across the surface and is depicted using contour lines, which in this example are shown as roughly ellipse-shaped. In the case of coaxial alignment of the eye 21 with respect to the central axis m of the patient interface 5, the incision surfaces C are also coaxial with respect to the central axis o of the eye 21 in the eye tissue 211, which extends through the apex of the eye 21. In an embodiment, the incision surface C comprises two partial incision surfaces which determine, or form, a lenticule in the eye tissue 211 which is asymmetrical with respect to the central axis m of the patient interface 52. In the following embodiments relating to the incision surface C and a rotated incision surface C', this formation of the lenticule by the partial incision surfaces of the incision surface C, or by the partial incision surfaces of the rotated incision surface C', are always to be understood even if this is not discussed further. The orientation of the incision surface C is indicated by an astigmatism axis a, which in Figure 5 is shown being at an angle with respect to a lateral axis LA of the eye 21 . Due to the rotation of the eye by the rotation angle **θ,** the control module 11 is configured to rotate the incision surface C about the rotation angle **θ** such that a rotated incision surface C' is incised in the eye.

**Figure 6b** shows an illustration of an incision surface C' in the eye tissue 211 of the eye 21 which has been rotated about the rotation angle (theta) from the unrotated incision surface C. In particular, the incision surface C as shown in Figure 5 has been rotated about the rotation angle to form the incision surface C' with an angle of astigmatism a'. **Figure 7** shows a flow diagram illustrating an exemplary series of steps for determining a rotation angle **θ** of an eye 21 of a person 2. The steps are performed while the person 2 is in a reclined position as illustrated in **Figure 3b****.** In particular, these steps are performed immediately prior to eye treatment.

In an embodiment, in preparatory step, the eye 21 of the person 2 is fixed using a patient interface 54 as shown in Figures 1 and 2.

In a step S1, the control module 2, in particular the processor 21, is configured to receive a reference image 31. For example, the processor 21 is configured to receive the reference image 31 from the memory 14 or from an auxiliary memory device via the communication interface 15. The reference image 31 is an image of the eye 21 of the person 2 taken prior to eye treatment, in particular by a diagnostic device 3 as illustrated in **Figure 3a****.**

In a step S2, the control module 2, in particular the processor 21, instructs the camera 12 to record a current image 121 of the eye 21. Depending on the embodiment, one or more current images 121 of the eye are recorded.

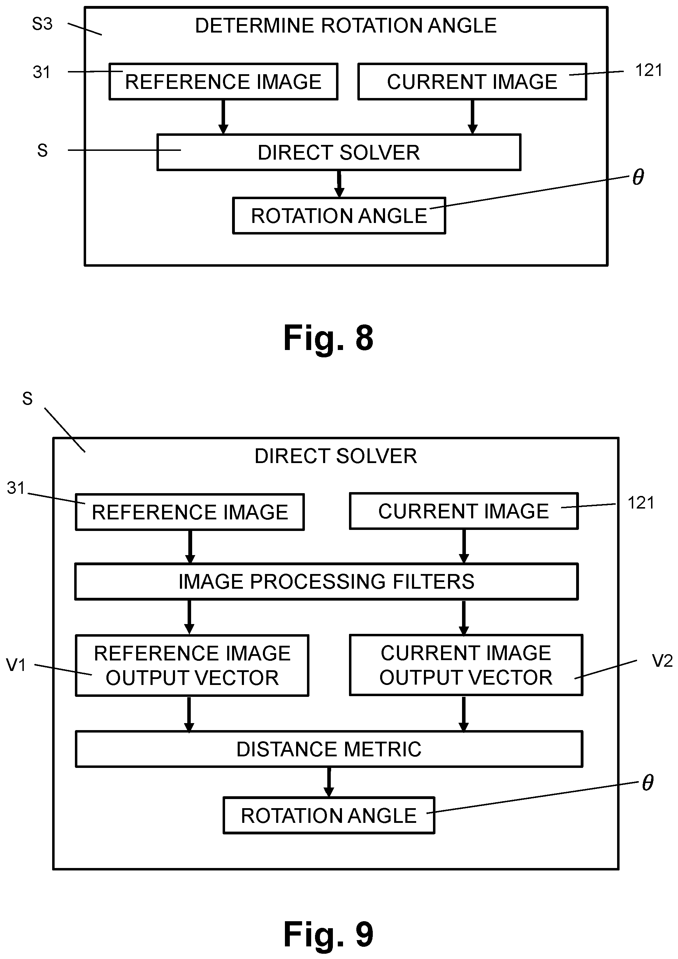

In a step S3, the processor 21 compares the current image 1 21 to the reference image 31 to determine a rotation angle **θ** of the eye 21 in current image 121 with respect to the reference image 31. In particular, the processor 21 uses a direct solver S for comparing the images as shown in **Figure 8****.** The direct solver S determines the rotation angle **θ** non-iteratively. This results in the processor 21 determining the rotation angle **θ** in a predictable time. Further, this ensures that the processor 21 is guaranteed to find a solution, which is not always the case with solvers using an iterative optimization approach.

By determining the rotation angle **θ** in a predictable time, the processor 21, in an embodiment, determines the rotation angle **θ** successively using successive current images 121 recorded by the camera, in real-time. This ensures that even if the person 2 shifts, rotates, or otherwise moves their head, the treatment pattern t is adjusted accordingly. Advantageously, this allows for treatment without the patient interface 52 being fixed to the surface of the eye 21.

Further, this may result in the processor 21 determining the rotation angle **θ** more quickly than iterative functions/algorithms and therefore results in a quicker overall treatment time as the person 2 does not have to lie down for as long. A quicker treatment is safer because the person 2 has less opportunity to move.

Depending on the embodiment, the direct solver S is implemented as a software application, an algorithm, and/or a function.

In an embodiment, the processor 21 is configured to display, on a display of the user interface of the ophthalmological treatment device 1, the reference image 31 and/or the current image 121 and the rotation angle **θ**. The processor 21 is configured to receive, via the user interface, user input from an eye treatment professional relating to the rotation angle **θ**. In particular, the user input comprises an indication to alter the determined rotation angle **θ**.

Depending on the embodiment, the processor 21 is configured to display the reference image 31 and the current image 121 simultaneously next to each other, preferably using a polar representation (as "unrolled" images), as explained below in more detail.

In an embodiment, the processor 21 is configured to display the reference image 31 and the current image 121 next to each other, e.g., one above the other, rendering the reference image 31 and/or the current image 121 such that both the reference image 31 and/or the current image 121 are both visible. In a preferred embodiment, a polar representation of the reference image 31 and a polar representation of the current image 121 are displayed. The polar representation maps a ring-shaped part of the images 31, 121 which relates to an area around the iris to a rectangular image, preferably of identical size.

The polar representation is generated, for example, by identifying a center point in the reference image 31 and a center point in the current image 121. The center points preferably correspond to the center of the eye, in particular the pupil, in the respective images 31, 121. The polar representation "unrolls" the images 31, 121, preferably by mapping a radial distance from the center point to a y-coordinate of the polar representation and mapping an azimuthal angle about the center point to an x-coordinate. Preferably, the polar representation of the reference image 31 and/or the current image 121 is displaced according to the rotation angle **θ** (the rotation angle being mapped to a displacement along the x-axis in the polar representation). If the rotation angle **θ** determined by the processor 21 is accurate, the displaced polar representation of the reference image 31 and/or the displaced polar representation of the current image 121 will align such that, at least in part, features of the reference image 31 and features of the current image 121 will line up, i.e. be present at the same horizontal positions.

Depending on the indication received as part of the user input, the rotation angle **θ** is manually updated to an updated rotation angle **θ**. The transformed reference image 31 and/or the transformed current image 121 are rendered according to the updated rotation angle **θ**. Thereby, the eye treatment professional is able to fine-tune the rotation angle **θ** determined by the processor 21 in an iterative and guided interaction. Once the rotation angle **θ** has been fine-tuned, the eye treatment professional can accept the updated rotation angle **θ**, which will be used for rotating the treatment pattern t to a determine a rotated treatment pattern t' as described below.

In an embodiment, the ophthalmological treatment device 1 is controlled using the rotation angle **θ**. In particular, the processor 21 is configured to rotate the treatment pattern t about the rotation angle **θ**, thereby resulting in a rotated treatment pattern t'. The ophthalmological treatment device 1 controls the laser L according to the rotated treatment pattern t' such that the laser L is directed onto one or more treatment points F. In an embodiment, the treatment pattern t, t' comprises a laser trajectory. The laser trajectory includes, for example, one or more continuous laser paths and/or one or more discrete treatment points F. The treatment pattern t, t' further includes, depending on the embodiment, one or more laser speeds, one or more laser spot sizes, and/or one or more laser powers.

**Figure 9** shows a block diagram illustrating an embodiment of the direct solver S. The direct solver S has a set of image processing filters configured to generate a reference image output vector V1 using the reference image 31 and a current image output vector V2 using the current image 121. The image processing filters include for example: affine and nonlinear transformations such as transformation of the ring-shaped iris region to polar coordinates, color adjustments such as histogram matching, edge-detection filters, gradient filters, Gabor filters, wavelets, and/or ridgelets. The output vectors V1, V2 are n-dimensional. The rotation angle **θ** is determined using the output vectors V1, V2 and a distance metric. The distance metric uses, for example, an L1 norm, an L2 norm, and/or a max norm. In an embodiment, the distance metric directly correlates with the rotation angle **θ** according to a pre-determined correlation function.

In an embodiment, the direct solver S, or a specific image pre-processing function, pre-processes the reference image 31 and/or the current image 121.

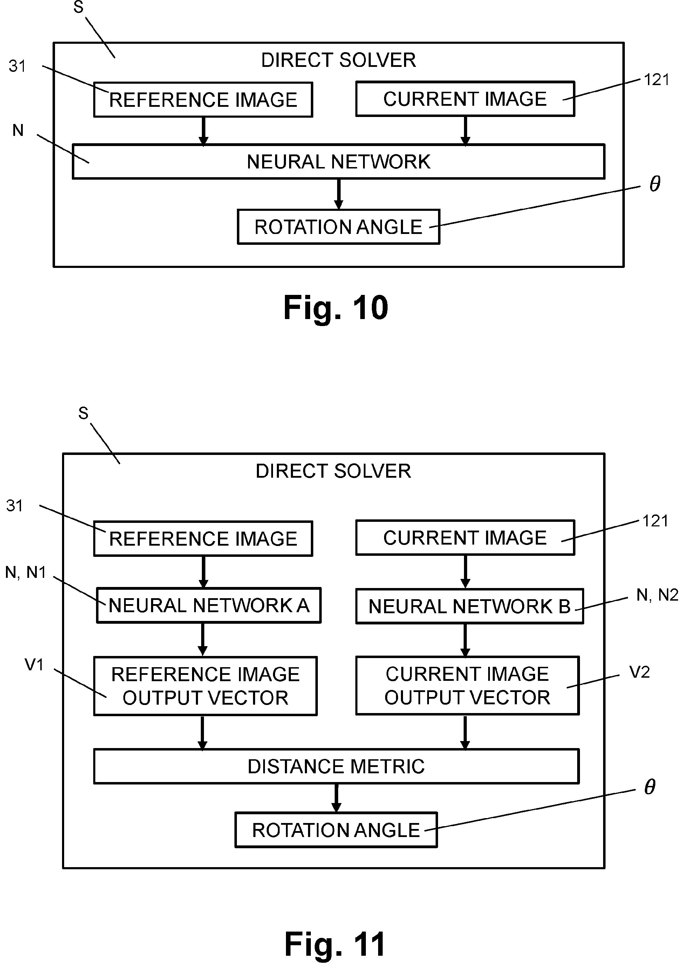

**Figure 10** shows a block diagram illustrating an embodiment of the direct solver S, the direct solver comprising a neural network N. The neural network N comprises one or more convolutional layers, one or more pooling layers, and one or more activation functions.

The neural network N is configured to receive both the reference image 31 and the current image 121 as inputs. The neural network N is further configured to output the rotation angle **θ**. The neural network N is configured to preprocess the inputs using a sequence of preprocessing steps. The preprocessing steps are designed to process the reference image 31 and the current image 121 such that particular characteristics of the images match. For example, the preprocessing steps comprise image transformations such as a transformation to polar coordinates and/or color adjustments such as histogram matching. The neural network N then contains convolutional layers, activation functions (ReLU), pooling operations, fully-connected layers, and/or skip connections. In particular, the neural network N comprises two final and dense fully-connected layers configured to directly output the rotation angle **θ**.

In an embodiment, the the neural network N includes a ResNet-34 architecture with two fully-connected layers at the end configured to directly output the rotation angle **θ**.

The neural network N is configured to determine the rotation angle **θ** by identifying non-local features in both the reference image 31 and the current image 121. Non-local features identified in both images 31, 121 are matched and a distance between them is determined. This distance is then used to determine the rotation angle **θ**.

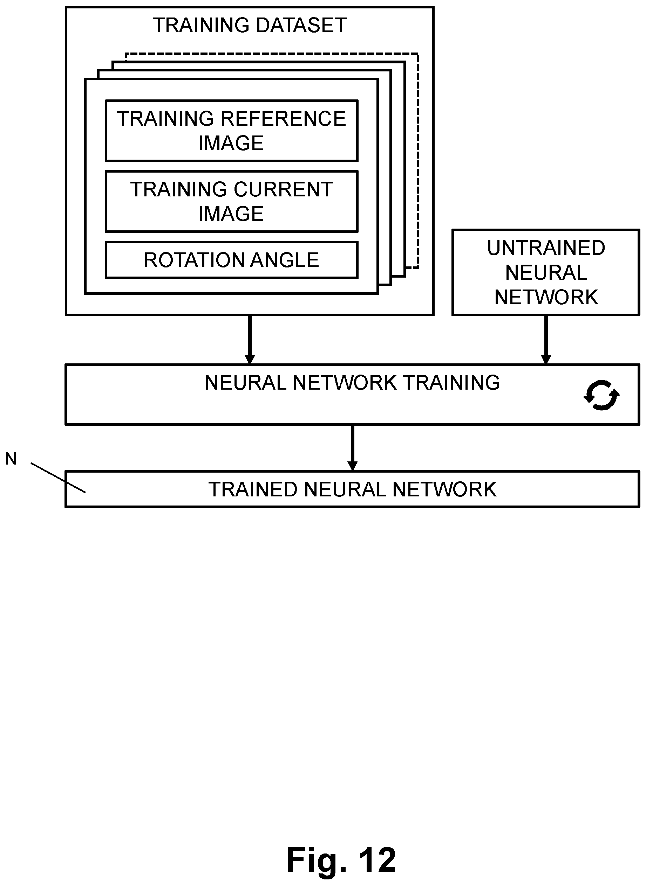

The neural network N is trained to output the rotation angle **θ**, for example, in the manner shown in **Figure 12****.**

In a preferred embodiment, the neural network N is executed by the processor 11 in a GPU and/or in a TPU for faster execution.

**Figure 11** shows a block diagram illustrating an embodiment of the direct solver S. The direct solver S comprises two neural networks N1, N2. Both neural networks N1, N2 have the identical architecture (i.e. the same layers and connections between the layers) and identical parameters, also known as a Siamese network. Each of the two neural networks N1, N2 is configured to generate an output vector from an input image. Specifically, a first neural network N1 is configured to generate a reference image output vector V1 using the reference image 31 and the second neural network N2 is configured to generate a current image output vector V2 using the current image 121.

The Siamese neural networks N1, N2 start with a chain of preprocessing steps as described above, which may include image transformations such as a transformation to polar coordinates and color adjustments such as histogram matching. The neural network architecture for the neural networks N1, N2 then contains convolutional layers, activation functions (ReLU), pooling operations, fully connected layers, and/or skip connections. One or two downstream dense and fully-connected layers, preferably connected directly to the output, perform a low-dimensional embedding (preferably n<100) to generate the reference image output vector V1 and the current image output vector V2.

The final dense and fully-connected layers are trained during the training phase such that the distance obtained using the distance metric is minimized for input image pairs where no rotation is present (between the images of the image pair) and maximized for input image pairs having a large rotation (between the images of the image pair). In a preferred embodiment, the neural networks N1, N2 include a ResNet-34 architecture with two downstream fully-connected layers, preferably directly upstream from the output. The reference image output vector V1 and the current image output vector V2 are then used to determine the rotation angle **θ** using the distance metric as described above.

**Figure 12** shows a block diagram illustrating an embodiment of how the neural network N, N1, N2 is trained.

The neural network N is initialized as an untrained neural network N of a particular architecture with random parameters, e.g. random weights and biases. The untrained neural network is trained using a training dataset comprising a large number, preferably in the order of 1000, more preferably at least 300 training reference images and associated training current images and training rotation angles. It is important that the training data set comprises a wide variety of lighting conditions as well as different eye shapes and iris colors to avoid any bias into the detector towards or against different ethnic groups. The training rotation angles are obtained from image pairs where the horizontal reference axis in the eye is marked before the patient is lying in supine position. The training dataset is then used to train the untrained neural network iteratively using supervised learning to generate the trained neural network N. In particular, the training dataset is segregated into a training subset, a test subset, and a validation subset. Data augmentation using rotation and/or mirroring of the input image pairs is used to enlarge the training set.

The neural network N can be successfully trained using, for example, the Adam optimizer using a learning rate of 3 · 10⁻⁴ . Training is helped significantly by injecting readily available pre-trained weights for a ResNet-34 architecture trained on the ImageNet database as a starting point.

The Siamese neural networks N1, N2 can be successfully trained using image pairs and a contrasting loss function.

Training of the neural networks N, N1, N2 takes place prior to treatment and is necessary only once. The trained neural networks N, N1, N2 is then stored in the memory 14 of the ophthalmological treatment device 1.

The above-described embodiments of the disclosure are exemplary and the person skilled in the art knows that at least some of the components and/or steps described in the embodiments above may be rearranged, omitted, or introduced into other embodiments without deviating from the scope of the present disclosure.

## Claims

1. An ophthalmological treatment device (1) comprising a processor (11) and a camera (12) for determining a rotation of an eye (21) of a person (2), the processor (11) configured to:
receive (S1) a reference image (31) of the eye (21), the reference image (31) having been recorded with the person (2) in an upright position by a separate diagnostic device (3);
record (S2), using the camera (12), a current image (121) of the eye (21), the current image (121) being recorded with the person (2) in a reclined position; and
determine (S3) a rotation angle (θ) of the eye (21) by comparing the reference image (31) to the current image (121) using a direct solver (S), **characterized in that** the direct solver (S) applies a pre-determined sequence of signal processing filters to both the entire reference image (31) and the entire current image (121),
the processor (11) configured to determine the rotation angle (θ) by:
generating a reference image output vector (V1) using the reference image (31) and the pre-determined sequence of signal processing filters;
generating a current image output vector (V2) using the current image (211) and the pre-determined sequence of signal processing filters; and
determining a distance (d) between the reference image output vector (V1) and the current image output vector (V2) using a distance metric.

2. The ophthalmological treatment device (1) of claim 1, wherein the processor (11) is further configured to rotate a treatment pattern (t) of a laser (13) about the rotation angle (θ), which treatment pattern (t) is configured for the eye (21) of the person (2).

3. The ophthalmological treatment device (1) of one of claims 1 or 2, wherein the direct solver (S) is configured to determine the rotation angle (θ) using a pre-defined number of computational operations, preferably within a pre-defined number of milliseconds.

4. The ophthalmological treatment device (1) of one of claims 1 to 3, wherein the processor (11) is configured to determine the rotation angle (θ) of the eye (21) by:
identifying one or more non-local features of the reference image (31) and non-local features of the current image (121), and
matching the one or more identified non-local features of the reference image (31) to the one or more identified non-local features of the current image (121), respectively.

5. The ophthalmological treatment device (1) of claim 1, wherein the pre-determined sequence of signal processing filters comprises one or more of: a convolutional operator, an activation function, or a pooling function.

6. The ophthalmological treatment device (1) of one of claims 1 or 5, wherein the pre-determined sequence of signal processing filters is part of a neural network (N).

7. The ophthalmological treatment device (1) of claim 6, comprising:
a first neural network (N1) and a second neural network (N2) both having identical architecture and parameters, the first neural network (N1) configured to receive the reference image (31) as an input and to generate a reference image output vector (V1), and the second neural network (N2) configured to receive the current image (121) as an input and to generate a current image output vector (V2),
wherein the processor (11) is configured to determine the rotation angle (θ) comprises using the reference image output vector (V1), the current image output vector (V2), and a distance metric.

8. The ophthalmological treatment device (1) of one of claims 1 to 7, wherein the processor (11) is further configured to pre-process the images, wherein pre-processing comprises one or more of:
detecting the iris of the eye (21) in the reference image (31) and/or the current image (121), in particular detecting an edge between the iris and the pupil of the eye (21);
detecting scleral blood vessels of the eye (21) in the reference image (31) and/or the current image (121);
detecting the retina of the eye (21) in the reference image (31) and/or the current image (121);
identifying a covered zone in the reference image, which covered zone is a part of the eye (21) covered by the eyelid;
unrolling the reference image (31) and/or the current image (121) using a polar transformation;
rescaling the reference image (31) and/or the current image (121) according to a detected pupil dilation in the reference image (31) and/or the current image (121);
image correcting the reference image (31) and/or the current image (121), in particular by matching an exposure, a contrast, and/or a color; or
resizing the reference image (31) and/or the current image (121) such that the reference image (31) and the current image (121) have a matching size.

9. A method for determining a rotation of an eye (21) of a person (2) comprising a processor (11) of an ophthalmological treatment device (1) performing the steps of:
receiving (S1) a reference image (31) of the eye (21), the reference image (31) having been recorded with the person (2) in an upright position by a separate diagnostic device (3);
recording (S2), using a camera (21) of the ophthalmological treatment device (1), a current image (121) of the eye (21), the current image (121) being recorded with the person (2) in a reclined position; and
determining (S3) a rotation angle (θ) of the eye (21) by comparing the reference image (31) to the current image (121), using a direct solver (S), **characterized in that** the direct solver (S) applies a pre-determined sequence of signal processing filters to both the entire reference image (31) and the entire current image (121),
the processor (11) configured to determine the rotation angle (θ) by:
generating a reference image output vector (V1) using the reference image (31) and the pre-determined sequence of signal processing filters;
generating a current image output vector (V2) using the current image (211) and the pre-determined sequence of signal processing filters; and
determining a distance (d) between the reference image output vector (V1) and the current image output vector (V2) using a distance metric.

10. The method of claim 9, further comprising rotating a treatment pattern (t) of a laser about the rotation angle (θ), which treatment pattern (t) is configured for the eye (21) of the person.

11. The method of claim 10, wherein the neural network is trained to determine the rotation angle (θ) using supervised learning and a training dataset, wherein the training dataset comprises a plurality of training reference images, a plurality of corresponding training current images, and a plurality of corresponding pre-defined rotation angles (θ), respectively.

12. The method of claim 10 or 11, comprising
a first neural network (N1) and a second neural network (N2) both having an identical architecture and identical parameters, the first neural network (N1) configured to receive the reference image (31) as an input and generating a reference image output vector (V1), and the second neural network (N2) configured to receive the current image (121) as an input and to generate a current image output vector (V2),
wherein determining the rotation angle (θ) comprises using the reference image output vector (V1), the current image output vector (V2), and a distance metric.

13. A computer program product comprising a non-transitory computer-readable medium having stored thereon computer program code for controlling a processor (11) of an ophthalmological treatment device (1) to:
receive (S1) a reference image (31) of the eye (21), the reference image (31) having been recorded with the person (2) in an upright position by a separate diagnostic device (3);
record (S2), using the camera (12), a current image (121) of the eye (21), the current image (121) being recorded with the person (2) in a reclined position; and
determine (S3) a rotation angle (θ) of the eye (21) by comparing the reference image (31) to the current image (121) using a direct solver (S), **characterized in that** the direct solver (S) applies a pre-determined sequence of signal processing filters to both the entire reference image (31) and the entire current image (121), and
determine the rotation angle (θ) by:
generating a reference image output vector (V1) using the reference image (31) and the pre-determined sequence of signal processing filters;
generating a current image output vector (V2) using the current image (211) and the pre-determined sequence of signal processing filters; and
determining a distance (d) between the reference image output vector (V1) and the current image output vector (V2) using a distance metric.

## Patentansprüche

1. Ophthalmologische Behandlungsvorrichtung (1) umfassend einen Prozessor (11) und eine Kamera (12), um eine Drehung eines Auges (21) einer Person (2) zu bestimmen, wobei der Prozessor (11) konfiguriert ist zum:
Empfangen (S1) eines Referenzbildes (31) des Auges (21), wobei das Referenzbild (31) mit der Person (2) in aufrechter Position durch eine separate Diagnosevorrichtung (3) aufgenommen wurde (3);
Aufnehmen (S2) eines aktuellen Bildes (121) des Auges (21) unter Verwendung der Kamera (12), wobei das aktuelle Bild (121) bei der Person (2) in einer liegenden Position aufgenommen wird; und
Bestimmen (S3) eines Drehwinkels (θ) des Auges (21) durch Vergleichen des Referenzbildes (31) mit dem aktuellen Bild (121) unter Verwendung eines direkten Solvers (S), **dadurch gekennzeichnet, dass** der direkte Solver (S) eine vorbestimmte Folge von Signalverarbeitungsfiltern sowohl auf das gesamte Referenzbild (31) als auch auf das gesamte aktuelle Bild (121) anwendet,
wobei der Prozessor (11) so konfiguriert ist, dass er den Drehwinkel (θ) bestimmt durch:
Erzeugen eines Referenzbild-Ausgabevektors (V1) unter Verwendung des Referenzbildes (31) und der vorbestimmten Folge von Signalverarbeitungsfiltern;
Erzeugen eines aktuellen Bild-Ausgabevektors (V2) unter Verwendung des aktuellen Bildes (211) und der vorbestimmten Folge von Signalverarbeitungsfiltern; und
Bestimmen eines Abstands (d) zwischen dem Referenzbild-Ausgabevektor (V1) und dem aktuellen Bild-Ausgabevektor (V2) unter Verwendung einer Abstandsmetrik.

2. Die ophthalmologische Behandlungsvorrichtung (1) nach Anspruch 1, wobei der Prozessor (11) ferner so konfiguriert ist, dass er ein Behandlungsmuster (t) eines Lasers (13) um den Drehwinkel (θ) dreht, wobei das Behandlungsmuster (t) für das Auge (21) der Person (2) konfiguriert ist.

3. Die ophthalmologische Behandlungsvorrichtung (1) nach einem der Ansprüche 1 oder 2, wobei der direkte Solver (S) so konfiguriert ist, dass er den Drehwinkel (θ) unter Verwendung einer vordefinierten Anzahl von Rechenoperationen, vorzugsweise innerhalb einer vordefinierten Anzahl von Millisekunden, bestimmt.

4. Die ophthalmologische Behandlungsvorrichtung (1) nach einem der Ansprüche 1 bis 3, wobei der Prozessor (11) so konfiguriert ist, dass er den Drehwinkel (θ) des Auges (21) bestimmt durch:
Identifizieren eines oder mehrerer nicht-lokaler Merkmale des Referenzbildes (31) und nicht-lokaler Merkmale des aktuellen Bildes (121) und
Abgleichen jeweils eines oder mehrere der identifizierten nicht-lokalen Merkmale des Referenzbildes (31) mit einem oder mehreren der identifizierten nicht-lokalen Merkmale des aktuellen Bildes (121).

5. Die ophthalmologische Behandlungsvorrichtung (1) nach Anspruch 1, wobei die vorbestimmte Folge von Signalverarbeitungsfiltern eines oder mehrere der folgenden Elemente umfasst: einen Faltungsoperator, eine Aktivierungsfunktion oder eine Pooling-Funktion.

6. Die ophthalmologische Behandlungsvorrichtung (1) nach einem der Ansprüche 1 oder 5, wobei die vorbestimmte Folge von Signalverarbeitungsfiltern Teil eines neuronalen Netzwerks (N) ist.

7. Die ophthalmologische Behandlungsvorrichtung (1) nach Anspruch 6, umfassend:
ein erstes neuronales Netzwerk (N1) und ein zweites neuronales Netzwerk (N2), die beide identische Architektur und Parameter aufweisen, wobei das erste neuronale Netzwerk (N1) so konfiguriert ist, dass es das Referenzbild (31) als Eingabe empfängt und einen Referenzbild-Ausgabevektor (V1) erzeugt, und das zweite neuronale Netzwerk (N2) so konfiguriert ist, dass es das aktuelle Bild (121) als Eingabe empfängt und einen aktuellen Bild-Ausgabevektor (V2) erzeugt,
wobei der Prozessor (11) so konfiguriert ist, dass er den Drehwinkel (θ) unter Verwendung des Referenzbild-Ausgabevektors (V1), des aktuellen Bild-Ausgabevektors (V2) und einer Abstandsmetrik bestimmt.

8. Die ophthalmologische Behandlungsvorrichtung (1) nach einem der Ansprüche 1 bis 7, wobei der Prozessor (11) ferner konfiguriert ist, die Bilder vorzuverarbeiten, wobei die Vorverarbeitung eines oder mehrere der folgenden Schritte umfasst:
Erfassen der Iris des Auges (21) im Referenzbild (31) und/oder im aktuellen Bild (121), insbesondere Erfassen einer Kante zwischen der Iris und der Pupille des Auges (21);
Erfassen von Sklerablutgefäßen des Auges (21) im Referenzbild (31) und/oder im aktuellen Bild (121);
Erfassen der Netzhaut des Auges (21) im Referenzbild (31) und/oder im aktuellen Bild (121);
Identifizieren eines verdeckten Bereichs im Referenzbild, wobei dieser verdeckte Bereich ein Teil des Auges (21) ist, der vom Augenlid verdeckt wird;
Entfalten des Referenzbildes (31) und/oder des aktuellen Bildes (121) unter Verwendung einer Polar-Transformation;
Neuskalieren des Referenzbildes (31) und/oder des aktuellen Bildes (121) gemäß einer im Referenzbild (31) und/oder im aktuellen Bild (121) erfassten Pupillenerweiterung;
Bildkorrektur des Referenzbildes (31) und/oder des aktuellen Bildes (121), insbesondere durch Anpassung von Belichtung, Kontrast und/oder Farbe; oder
Anpassen der Größe des Referenzbildes (31) und/oder des aktuellen Bildes (121) derart, dass das Referenzbild (31) und das aktuelle Bild (121) eine übereinstimmende Größe aufweisen.

9. Verfahren zur Bestimmung einer Drehung eines Auges (21) einer Person (2), wobei ein Prozessor (11) einer ophthalmologischen Behandlungsvorrichtung (1) die folgenden Schritte ausführt:
Empfangen (S1) eines Referenzbildes (31) des Auges (21), wobei das Referenzbild (31) mit der Person (2) in aufrechter Position durch eine separate Diagnosevorrichtung (3) aufgenommen wurde;
Aufnehmen (S2) eines aktuellen Bildes (121) des Auges (21) unter Verwendung einer Kamera (21), wobei das aktuelle Bild (121) bei der Person (2) in einer liegenden Position aufgenommen wird ; und
Bestimmen (S3) eines Drehwinkels (θ) des Auges (21) durch Vergleichen des Referenzbildes (31) mit dem aktuellen Bild (121) unter Verwendung eines direkten Solvers (S), **dadurch gekennzeichnet, dass** der direkte Solver (S) eine vorbestimmte Folge von Signalverarbeitungsfiltern sowohl auf das gesamte Referenzbild (31) als auch auf das gesamte aktuelle Bild (121) anwendet,
wobei der Prozessor (11) so konfiguriert ist, dass er den Drehwinkel (θ) bestimmt durch:
Erzeugen eines Referenzbild-Ausgabevektors (V1) unter Verwendung des Referenzbildes (31) und der vorbestimmten Folge von Signalverarbeitungsfiltern;
Erzeugen eines aktuellen Bild-Ausgabevektors (V2) unter Verwendung des aktuellen Bildes (211) und der vorbestimmten Folge von Signalverarbeitungsfiltern; und
Bestimmen eines Abstands (d) zwischen dem Referenzbild-Ausgabevektor (V1) und dem aktuellen Bild-Ausgabevektor (V2) unter Verwendung einer Abstandsmetrik.

10. Das Verfahren nach Anspruch 9, das ferner das Drehen eines Behandlungsmusters (t) eines Lasers um den Drehwinkel (θ) umfasst, wobei das Behandlungsmuster (t) für das Auge (21) der Person konfiguriert ist.

11. Das Verfahren nach Anspruch 10, wobei das neuronale Netzwerk darauf trainiert ist, den Drehwinkel (θ) unter Verwendung von überwachtem Lernen und eines Trainingsdatensatzes zu bestimmen, wobei der Trainingsdatensatz jeweils eine Vielzahl von Trainingsreferenzbildern, eine Vielzahl von entsprechenden Trainingsaktuellbildern und eine Vielzahl von entsprechenden vordefinierten Drehwinkeln (θ) umfasst.

12. Das Verfahren nach Anspruch 10 oder 11, umfassend
ein erstes neuronales Netzwerk (N1) und ein zweites neuronales Netzwerk (N2), die beide eine identische Architektur und identische Parameter aufweisen, wobei das erste neuronale Netzwerk (N1) so konfiguriert ist, dass es das Referenzbild (31) als Eingabe empfängt und einen Referenzbild-Ausgabevektor (V1) erzeugt, und das zweite neuronale Netzwerk (N2) so konfiguriert ist, dass es das aktuelle Bild (121) als Eingabe empfängt und einen aktuellen Bild-Ausgabevektor (V2) erzeugt,
wobei das Bestimmen des Drehwinkels ( θ ) die Verwendung des Referenzbild-Ausgabevektors (V1), des aktuellen Bild-Ausgabevektors (V2) und einer Abstandsmetrik umfasst.

13. Ein Computerprogrammprodukt, das ein nichtflüchtiges, computerlesbares Medium umfasst, auf dem Computerprogrammcode gespeichert ist, um einen Prozessor (11) einer ophthalmologischen Behandlungsvorrichtung (1) zu steuern für das:
Empfangen (S1) eines Referenzbildes (31) des Auges (21), wobei das Referenzbild (31) mit der Person (2) in aufrechter Position durch eine separate Diagnosevorrichtung (3) aufgenommen wurde;
Aufnehmen (S2) eines aktuellen Bildes (121) des Auges (21) unter Verwendung der Kamera (12), wobei das aktuelle Bild (121) bei der Person (2) in einer liegenden Position aufgenommen wird; und
Bestimmen (S3) eines Drehwinkels (θ) des Auges (21) durch Vergleichen des Referenzbildes (31) mit dem aktuellen Bild (121) unter Verwendung eines direkten Solvers (S), **dadurch gekennzeichnet, dass** der direkte Solver (S) eine vorbestimmte Folge von Signalverarbeitungsfiltern sowohl auf das gesamte Referenzbild (31) als auch auf das gesamte aktuelle Bild (121) anwendet, und
Bestimmung des Drehwinkels (θ) durch:
Erzeugen eines Referenzbild-Ausgabevektors (V1) durch Verwendung des Referenzbildes (31) und der vorbestimmten Folge von Signalverarbeitungsfiltern;
Erzeugen eines aktuellen Bild-Ausgabevektors (V2) unter Verwendung des aktuellen Bildes (211) und der vorbestimmten Folge von Signalverarbeitungsfiltern; und
Bestimmen eines Abstands (d) zwischen dem Referenzbild-Ausgabevektor (V1) und dem aktuellen Bild-Ausgabevektor (V2) unter Verwendung einer Abstandsmetrik.

## Revendications

1. Dispositif de traitement ophtalmologique (1) comprenant un processeur (11) et une caméra (12) pour déterminer la rotation d'un œil (21) d'une personne (2), le processeur (11) étant configuré pour :
recevoir (S1) une image de référence (31) de l'œil (21), l'image de référence (31) ayant été enregistrée alors que la personne (2) se trouvait en position debout à l'aide d'un dispositif de diagnostic distinct (3) ;
enregistrer (S2), à l'aide de la caméra (12), une image actuelle (121) de l'œil (21), l'image actuelle (121) étant enregistrée alors que la personne (2) est en position allongée ; et
déterminer (S3) un angle de rotation (θ) de l'œil (21) en comparant l'image de référence (31) à l'image actuelle (121) à l'aide d'un solveur direct (S), **caractérisé en ce que** le solveur direct (S) applique une séquence prédéterminée de filtres de traitement du signal à la fois à l'ensemble de l'image de référence (31) et à l'ensemble de l'image actuelle (121),
le processeur (11) étant configuré pour déterminer l'angle de rotation (θ) en:
générer un vecteur de sortie d'image de référence (V1) à l'aide de l'image de référence (31) et de la séquence prédéterminée de filtres de traitement du signal ;
générer un vecteur de sortie d'image actuelle (V2) à l'aide de l'image actuelle (211) et de la séquence prédéterminée de filtres de traitement du signal ; et
déterminer une distance (d) entre le vecteur de sortie d'image de référence (V1) et le vecteur de sortie d'image actuelle (V2) à l'aide d'une métrique de distance.

2. Dispositif de traitement ophtalmologique (1) selon la revendication 1, dans lequel le processeur (11) est en outre configuré pour faire tourner un motif de traitement (t) d'un laser (13) d'un angle de rotation ( θ ), lequel motif de traitement (t) est configuré pour l'œil (21) de la personne (2).

3. Dispositif de traitement ophtalmologique (1) selon l'une des revendications 1 ou 2, dans lequel le solveur direct (S) est configuré pour déterminer l'angle de rotation (θ) à l'aide d'un nombre prédéfini d'opérations de calcul, de préférence en un nombre prédéfini de millisecondes.

4. Dispositif de traitement ophtalmologique (1) selon l'une des revendications 1 à 3, dans lequel le processeur (11) est configuré pour déterminer l'angle de rotation (θ) de l'œil (21) en :
identifiant une ou plusieurs caractéristiques non locales de l'image de référence (31) et des caractéristiques non locales de l'image actuelle (121), et
en faisant correspondre respectivement la ou les caractéristiques non locales identifiées de l'image de référence (31) à la ou aux caractéristiques non locales identifiées de l'image actuelle (121).

5. Dispositif de traitement ophtalmologique (1) selon la revendication 1, dans lequel la séquence prédéterminée de filtres de traitement du signal comprend un ou plusieurs des éléments suivants : un opérateur de convolution, une fonction d'activation ou une fonction de regroupement.

6. Dispositif de traitement ophtalmologique (1) selon l'une des revendications 1 ou 5, dans lequel la séquence prédéterminée de filtres de traitement du signal fait partie d'un réseau neuronal (N).

7. Dispositif de traitement ophtalmologique (1) selon la revendication 6, comprenant :
un premier réseau neuronal (N1) et un deuxième réseau neuronal (N2) ayant tous deux une architecture et des paramètres identiques, le premier réseau neuronal (N1) étant configuré pour recevoir l'image de référence (31) en entrée et pour générer un vecteur de sortie d'image de référence (V1), et le deuxième réseau neuronal (N2) étant configuré pour recevoir l'image actuelle (121) en entrée et pour générer un vecteur de sortie d'image actuelle (V2),
dans lequel le processeur (11) est configuré pour déterminer l'angle de rotation (θ) en comprenant le vecteur de sortie d'image de référence (V1), le vecteur de sortie d'image actuelle (V2) et une métrique de distance.

8. Dispositif de traitement ophtalmologique (1) selon l'une des revendications 1 à 7, dans lequel le processeur (11) est en outre configuré pour prétraiter les images, le prétraitement comprenant une ou plusieurs des opérations suivantes:
détecter l'iris de l'œil (21) dans l'image de référence (31) et/ou l'image actuelle (121), en particulier détecter un bord entre l'iris et la pupille de l'œil (21) ;
détecter les vaisseaux sanguins scléraux de l'œil (21) dans l'image de référence (31) et/ou l'image actuelle (121) ;
détecter la rétine de l'œil (21) dans l'image de référence (31) et/ou l'image actuelle (121) ;
identifier une zone couverte dans l'image de référence, laquelle zone couverte est une partie de l'œil (21) recouverte par la paupière ;
déplier l'image de référence (31) et/ou l'image actuelle (121) à l'aide d'une transformation polaire ;
redimensionner l'image de référence (31) et/ou l'image actuelle (121) en fonction d'une dilatation de la pupille détectée dans l'image de référence (31) et/ou l'image actuelle (121) ;
corriger l'image de référence (31) et/ou l'image actuelle (121), notamment en ajustant l'exposition, le contraste et/ou la couleur ; ou
redimensionner l'image de référence (31) et/ou l'image actuelle (121) de manière à ce que l'image de référence (31) et l'image actuelle (121) aient une taille identique.

9. Procédé pour déterminer une rotation d'un œil (21) d'une personne (2), comprenant un processeur (11) d'un dispositif de traitement ophtalmologique (1) effectuant les étapes suivantes :
recevoir (S1) une image de référence (31) de l'œil (21), l'image de référence (31) ayant été enregistrée avec la personne (2) en position verticale par un dispositif de diagnostic séparé (3) ;
enregistrer (S2), à l'aide d'une caméra (21) du dispositif de traitement ophtalmologique (1), une image actuelle (121) de l'œil (21), l'image actuelle (121) étant enregistrée alors que la personne (2) est en position allongée ; et
déterminer (S3) un angle de rotation (θ) de l'œil (21) en comparant l'image de référence (31) à l'image actuelle (121), à l'aide d'un solveur direct (S),
**caractérisé en ce que** le solveur direct (S) applique une séquence prédéterminée de filtres de traitement du signal à la fois à l'ensemble de l'image de référence (31) et à l'ensemble de l'image actuelle (121),
le processeur (11) étant configuré pour déterminer l'angle de rotation (θ) en:
générer un vecteur de sortie d'image de référence (V1) à l'aide de l'image de référence (31) et de la séquence prédéterminée de filtres de traitement du signal ;
générer un vecteur de sortie d'image actuelle (V2) à l'aide de l'image actuelle (211) et de la séquence prédéterminée de filtres de traitement du signal ; et
déterminer une distance (d) entre le vecteur de sortie d'image de référence (V1) et le vecteur de sortie d'image actuelle (V2) à l'aide d'une métrique de distance.

10. Procédé selon la revendication 9, comprenant en outre la rotation d'un motif de traitement (t) d'un laser selon l'angle de rotation (θ), lequel motif de traitement (t) est configuré pour l'œil (21) de la personne.

11. Procédé selon la revendication 10, dans lequel le réseau neuronal est entraîné pour déterminer l'angle de rotation (θ) à l'aide d'un apprentissage supervisé et d'un ensemble de données d'entraînement, l'ensemble de données d'entraînement comprenant respectivement une pluralité d'images de référence d'entraînement, une pluralité d'images actuelles d'entraînement correspondantes et une pluralité d'angles de rotation prédéfinis correspondants (θ).

12. Procédé selon la revendication 10 ou 11, comprenant
un premier réseau neuronal (N1) et un deuxième réseau neuronal (N2) ayant tous deux une architecture et des paramètres identiques, le premier réseau neuronal (N1) étant configuré pour recevoir l'image de référence (31) en entrée et générer un vecteur de sortie d'image de référence (V1), et le deuxième réseau neuronal (N2) étant configuré pour recevoir l'image actuelle (121) en entrée et générer un vecteur de sortie d'image actuelle (V2),
dans lequel la détermination de l'angle de rotation (θ) comprend l'utilisation du vecteur de sortie d'image de référence (V1), du vecteur de sortie d'image actuelle (V2) et d'une métrique de distance.

13. Produit logiciel comprenant un support non transitoire lisible par ordinateur sur lequel est stocké un code de programme informatique destiné à commander un processeur (11) d'un dispositif de traitement ophtalmologique (1) afin de :
recevoir (S1) une image de référence (31) de l'œil (21), l'image de référence (31) ayant été enregistrée alors que la personne (2) se trouvait en position verticale par un dispositif de diagnostic distinct (3) ;
enregistrer (S2), à l'aide de la caméra (12), une image actuelle (121) de l'œil (21), l'image actuelle (121) étant enregistrée alors que la personne (2) est en position allongée ; et
déterminer (S3) un angle de rotation (θ) de l'œil (21) en comparant l'image de référence (31) à l'image actuelle (121) à l'aide d'un solveur direct (S), **caractérisé en ce que** le solveur direct (S) applique une séquence prédéterminée de filtres de traitement du signal à la fois à l'ensemble de l'image de référence (31) et à l'ensemble de l'image actuelle (121), et
déterminer l'angle de rotation (θ) en :
générer un vecteur de sortie d'image de référence (V1) à l'aide de l'image de référence (31) et de la séquence prédéterminée de filtres de traitement du signal ;
générer un vecteur de sortie d'image actuelle (V2) à l'aide de l'image actuelle (211) et de la séquence prédéterminée de filtres de traitement du signal ; et
déterminer une distance (d) entre le vecteur de sortie d'image de référence (V1) et le vecteur de sortie d'image actuelle (V2) à l'aide d'une métrique de distance .
